# FASCICULE DE BREVET EUROPEEN

(11) **EP 1 195 158 B1**
(45) Date de publication et mention de la délivrance du brevet: **30.07.2014**
(21) Numéro de dépôt: 01402601.7
(22) Date de dépôt: 09.10.2001
(51) Int. Cl.: A61K 9/12, A61K 31/192, B65D 83/14, A61M 11/02

(54) **Compositions pharmaceutiques sous forme de pulvérisation contenant du kétoprofène**
Verspühbare pharmazeutische Zusammensetzungen enthaltend Ketoprofen
Sprayable pharmaceutical compositions containing ketoprofen

(30) Priorité: 09.10.2000 FR 0012881
(43) Date de publication de la demande: 10.04.2002
(73) Titulaire: Menarini France S.A., 94550 Chevilly Larue (FR)
(72) Inventeur: Bertrand, Jean-Christophe, 92290 Chatenay Malabry (FR); Dubois, Martine, 75116 Paris (FR)
(74) Mandataire: Hubert, Philippe

(56) Documents cités:
- EP-A- 0 756 870
- EP-A1- 0 502 502
- EP-A1- 0 698 393
- WO-A2-99/20259
- FR-A- 2 076 684
- US-A- 4 704 406
- S PORZIO: 'EFFICACY OF A NEW TOPICAL GEL-SPRAY FORMULATION OF KETOPROFEN LYSINE SALT IN THE RAT: PERCUTANEOUS PERMEATIONIN VITROANDIN VIVOAND PHARMACOLOGICAL ACTIVITY' PHARMACOLOGICAL RESEARCH vol. 37, no. 1, 01 Janvier 1998, pages 41 - 47, XP055078887 DOI: 10.1006/phrs.1997.0260 ISSN: 1043-6618
- "VIDAL 2007 - Ketum Gel et Voltarène Emulgel", 2007

## Description

La présente invention se rapporte au domaine de la chimie thérapeutique et plus particulièrement au domaine de la formulation pharmaceutique.

Elle concerne plus spécifiquement des formes pharmaceutiques liquides contenant un agent anti-inflammatoire, susceptibles d'être projetées par pulvérisation sur la zone ou la région, nécessitant l'application d'un agent anti-inflammatoire.

L'invention concerne spécifiquement de nouvelles préparations pharmaceutiques à action anti-inflammatoire, caractérisées en ce qu'elles contiennent un principe actif anti-inflammatoire dissout ou dispersé dans un solvant organique volatil, en ce que la phase organique ainsi obtenue est additionnée d'un agent visqueux, puis ajustée à pH neutre et en ce que la composition répartie sous pression de gaz inerte, en flacons pressurisés surmontés d'une vanne de distribution sertie sur le flacon pressurisé, est destinée à être appliquée sur la peau.

Les compositions pharmaceutiques liquides contenant un agent anti-inflammatoire selon l'invention sont caractérisées en ce qu'elles renferment du kétoprofène sous forme acide ou sous forme salifiée, dispersée dans un alcanol ayant de 1 à 6 atomes de carbone dilué par de l'eau, un polyéthylène glycol 400 ou 600 et un agent d'ajustement à pH neutre et en ce que les compositions sont réparties sous pression de gaz inerte en flacons pressurisés.

L'application de produits anti-inflammatoires s'effectue en général sous forme d'une crème, d'un gel, d'un liniment ou d'une pommade sur la zone enflammée.

L'application de telles préparations ne nécessite pas de modalités particulières si ce n'est le massage de la région pour assurer un bon étalement de la composition et en outre pour en assurer la pénétration à travers l'épiderme. Cette pénétration nécessite en outre l'addition d'un promoteur qui facilite le passage de la préparation à travers l'épiderme. Sans cela, une fraction très faible de la préparation passe à travers la peau et il en résulte une perte considérable de préparation.

L'étalement d'une pommade ou d'une crème sur une zone enflammée peut être douloureux en raison de la rubéfaction de cette zone et de la manipulation mécanique plus ou moins énergique rendue nécessaire.

On connaît la demande de brevet européen EP-0756870 qui décrit une formulation pharmaceutique vaporisable comprenant comme principe actif un analgésique/anti-rhumatique tel que le kétoprofène pour le traitement des maladies rhumatiques ou traumatiques. Cette formulation contient, en plus du principe actif, un agent rubéfiant, des véhicules, un agent de pénétration, un agent filmogène ainsi que des agents neutralisants. Dans cette demande, le but est de fournir des agents capables de promouvoir la pénétration des médicaments à travers la peau en se basant sur l'amélioration de l'afflux sanguin et une meilleure pénétration.

On connaît également le brevet US-4704406 qui concerne des préparations pharmaceutiques vaporisables pour application topique. Ces préparations contiennent comme principe actif, un acide arylalcanoïque pouvant être du kétoprofène, et comme solvant, un mélange d'au moins un solvant volatile et au moins un solvant non volatile. L'efficacité est obtenue quand le principe actif est dissous dans un mélange de solvant volatile et non volatile dont le ratio est optimisé. Ces préparations peuvent être utilisées dans le traitement externe des maladies rhumatiques ou inflammatoires.

La demande de brevet européen EP-0698393 décrit un agent solubilisant, le 3-ℓ-menthoxypropane-1,2-diol, pour principes actifs pharmaceutiques utilisés dans des préparations percutanées absorbables. L'objectif est de fournir un agent solubilisant montrant d'excellentes capacités de solubilisation du principe actif et une excellente innocuité, stabilité et compatibilité. Ainsi, ces préparations peuvent se présenter sous forme d'aérosol comprenant, en plus du solubilisant et du principe actif, comme solvant au moins un alcool et de l'eau et comme gaz propulseur du diméthyle éther et/ou du gaz de pétrole liquéfié.

La demande de brevet européen EP-0502502 décrit une composition pharmaceutique topique sous forme de mousse qui contient comme principe actif un sel de lysine de kétoprofène. Cette mousse est émulsifiée au moyen d'un mélange de gaz et peut également contenir un tensioactif, un émulsifiant ionique ou non ionique, une substance émolliente, un épaississant et optionnellement du parfum et des substances bactériostatiques. Cette composition vise à permettre de ne pas avoir à administrer des concentrations élevées de principe actif.

Enfin, S. Porzio décrit dans Pharmacological Research, 37(1), 1998, pages 41-47, une comparaison entre l'efficacité d'un gel-spray de kétoprofène lysine et du gel Profénid. Le gel-spray est un système aqueux avec des excipients à caractère tixotropique qui ne contient pas d'alcool et est appliqué sur la peau avec une pompe mécanique. Le gel Profénid contient du kétoprofène, du carbomer, de l'éthanolamine, de l'éthanol, de l'eau, ainsi que de l'huile de lavande. Le gel-spray conduit à une meilleure absorption du kétoprofène par rapport au gel Profénid.

C'est pourquoi il est apparu souhaitable de procéder différemment à l'administration, par voie topique, des préparations destinées à être appliquées sur des zones articulaires ou tendineuses, douloureuses et/ou enflammées sans qu'il soit nécessaire d'avoir recours à des manipulations physiques.

En conséquence il s'est avéré particulièrement intéressant de réaliser des compositions pressurisées à base d'agent anti-inflammatoire et de les pulvériser sur la zone à traiter à une pression suffisante pour que la préparation pulvérisée s'étale sur la totalité de la zone à traiter et pénètre au maximum dans le derme.

L'invention a pour objet des compositions pharmaceutiques liquides contenant un agent anti-inflammatoire, caractérisées en ce qu'elles renferment du kétoprofène sous forme acide ou sous forme salifiée, dispersée dans un alcanol ayant de 1 à 6 atomes de carbone dilué par de l'eau, un polyéthylène glycol 400 ou 600 et un agent d'ajustement à pH neutre et en ce que les compositions sont réparties sous pression de gaz inerte en flacons pressurisés.

L'agent anti-inflammatoire selon l'invention est un acide arylacétique et en particulier le Kétoprofène sous forme (R), sous forme (S) ou sous forme (RS).

L'agent anti-inflammatoire peut être également sous forme de sel et notamment de sel de métal alcalin ou alcalinoterreux comme un sel de sodium, de potassium, de lithium, de magnésium ou de calcium. Il peut être également sous forme de sel de base organique comme une amine, un aminoalcool, un aminoacide basique ou un sel d'ammonium quaternaire.

Parmi ceux-ci on citera tout particulièrement les sels de diethylamine, de diethylaminoéthanol, de triethanolamine, de N-méthyl glucamine, de trométhamine, de trométhamol, de lysine, d'arginine, de N-méthyl benzylamine, de pipéridino éthanol, de pyrolidino éthanol, de N-méthyl pipérazine ou bien encore de cétyl pyridinium.

Les sels avec une base organique présentent l'avantage d'être plus solubles dans les solvants organiques que les sels avec une base minérale et peuvent ainsi être plus aisément dissous en milieu organique. On peut également réaliser des solutions plus concentrées.

Le solvant organique utilisé pour disperser ou dissoudre le principe actif est un alcanol et de préférence un alcanol ayant de 1 à 6 atomes de carbone. On pourra citer à cet égard le méthanol, l'éthanol, le propanol, l'isopropanol, les n et i-butanols, le terbutanol, le néopentanol ou le n-hexanol. Ces alkanols sont dilués par addition d'eau de manière à réaliser des véhicules hydroalcooliques comme de l'alcool éthylique à 50 % ou à 95 %, ou de l'isopropanol ou du terbutanol à 20 % d'eau.

Le soluté peut être avantageusement additionné d'un agent de pénétration comme par exemple le solketal, l'azone, le méthylal, le monocaprylate de propylèneglycol (Caprisol 90), l'octyl-2 dodecanol ou le 2-n-nonyl 1,3-dioxolane. La proportion d'agent de pénétration varie de 3 à 8 % de la composition totale et d'une manière davantage préférée de 4 à 6 % en poids.

Le véhicule alcoolique est en outre additionné d'un agent épaississant, qui est un poly éthylène glycol et notamment un PEG 400 ou un PEG 600.

Le pH du véhicule est légèrement acide (valeur 4,5 - 5). On l'ajuste aux alentours de 5 à 5,5 par addition de la quantité minimale d'hydroxyde de sodium, tout en respectant les zones de pH compatibles avec la stabilité du principe actif et la durabilité de la suspension, en fonction des caractéristiques chimiques des autres ingrédients. En particulier l'utilisation de sels de Kétoprofène très basiques nécessite l'ajustement du pH à l'aide d'acides minéraux ou organiques.

Le soluté à disperser peut avantageusement être additionné d'un parfum comme un produit aromatisant ou odoriférant. On pourra citer à cet égard l'essence de roses, l'essence de neroli, l'essence de petit-grain, l'essence de lavande, l'essence de lavandin, l'essence de thym, l'essence d'eucalyptus ou l'essence d'ylang-ylang, ainsi que des produits synthétiques comme la vetivone, la damascone, l'irone ou les ionones.

Le produit aromatisant ou odoriférant est additionné en quantité de 0,05 à 1 % de la composition totale. Une quantité préférée s'échelonne de 0,1 à 0,6 %.

La divulgation concerne également un dispositif de pulvérisation pour préparations pharmaceutiques selon l'invention qui consiste à répartir ladite solution dans des bidons propres à la pressurisation, à saturer l'atmosphère existant au-dessus de la phase liquide, par injection d'un gaz inerte non miscible à l'eau et à l'alcanol, à placer au sommet du bidon une valve obturatrice comportant un bouton-poussoir actionnant ladite valve.

L'angle de pulvérisation varie entre 40 et 50°. Il permet de couvrir à chaque projection une zone plus ou moins étalée. L'évaporation rapide du solvant organique assure un moindre écoulement de la préparation sur l'épiderme et une meilleure adhésion sur les zones traitées.

La valve sert également de moyen de dosage. Une poussée sur le bouton-poussoir permet la délivrance d'un volume de solution variant de 0,2 à 0,8 ml et de préférence de 0,4 à 0,6 ml. Le volume projeté dépend de la pression gazeuse régnant dans le bidon pressurisé. La valve utilisée de préférence est celle commercialisée par la société Sofab.

La concentration en principe actif dans la solution alcanolique s'échelonne de 1 à 10 % et de préférence de 2 à 5 %. De cette façon, chaque poussée permet la délivrance de 0,02 g à 0,8 g de principe actif.

La pompe doseuse qui permet la pulvérisation comprend, outre la solution hydroalkanolique d'agent anti-inflammatoire, un corps plongeant dans le liquide à pulvériser, une poche doseuse surmontée d'un clapet de mesure, un corps de soutien de la poche doseuse, des coupelles symétriques en aluminium sur lesquelles reposent, symétriquement disposés, une partie obturatrice serrée contre la coupelle par un joint interne, ainsi que deux amortisseurs en élastomère, disposés de part et d'autre de l'axe de l'obturateur, et le corps du gicleur mobile dans le sens vertical maintenu en place par des ressorts, sur lequel vient se fixer la buse de pulvérisation.

La valve est terminée par un embout légèrement arrondi sur lequel vient s'emboîter la valve doseuse.

Les bidons de remplissage sont remplis sous atmosphère d'un gaz inerte comme l'azote, l'hélium, l'argon, le protoxyde d'azote, le gaz carbonique ou un hydrocarbure volatil.

La pression exercée sur la valve doseuse fait descendre la tige du gicleur en contact avec le clapet de mesure de sorte que le liquide sous pression dans le tube plongeur pénètre dans le gicleur et est évacué par la valve dès que la pression se relâche.

Le gicleur est en copolymère acétal. Le tube plongeur est en polyéthylène. Le tube plongeur présente une longueur de 4 à 5 mm et un diamètre de 0,15 à 0,20 mm.

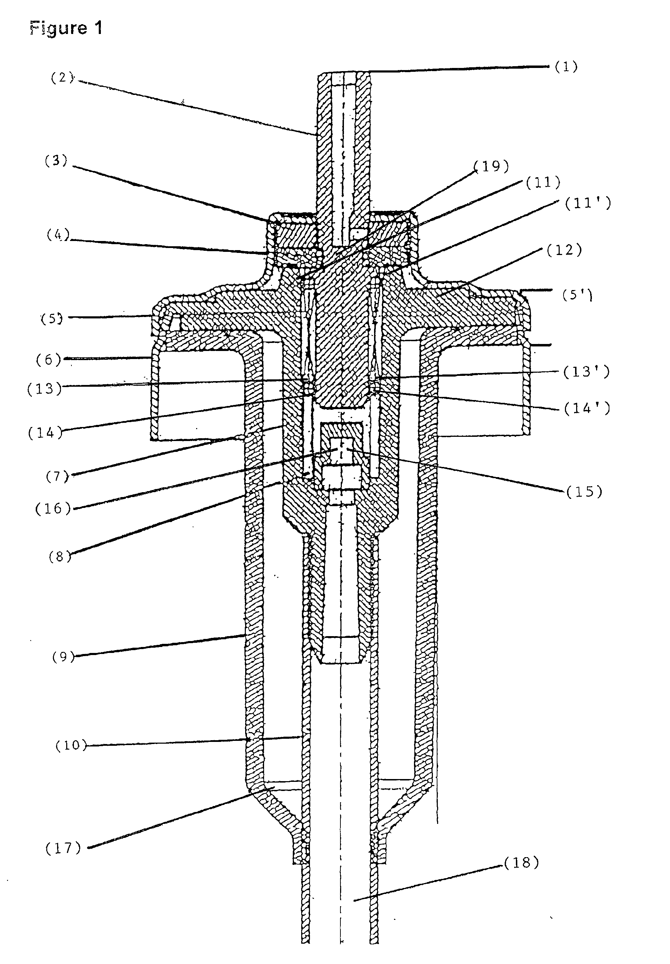

La figure 1 annexée montre une coupe du bidon pressurisé comportant la tige du gicleur (2), l'extrémité de la tige du gicleur (1), les deux amortisseurs symétriques (3) (3'), les supports de base de la tige du gicleur (4) encastrés dans le corps du gicleur (2) par un évidement (19) et reposant sur deux pivots (11) (11') montés sur un capot d'étanchéité (12). Ils sont assujettis au clapet de mesure (8) par deux ressorts (13) (13') fixés à leur extrémité inférieure sur un rebord (14) (14') du clapet. Le clapet (15) comporte en son centre un orifice (16) par lequel monte le liquide sous pression, contenu dans le bidon pressurisé (non figuré) par l'intermédiaire de la poche doseuse (9) en élastomère, fermée par un joint d'étanchéité (17) à sa base. Le tube plongeur (18) assure la montée du liquide (13) dans la poche doseuse (9) puis dans le corps du clapet (15).

Après relâchement de la valve doseuse, le liquide redescend par le tube plongeur (18) dans le bidon pressurisé.

Les exemples suivants illustrent l'invention.

### EXEMPLE 1

### Soluté de Kétoprofène

On dissout 5g de Kétoprofène dans 80g d'éthanol à 95-96 %. On ajoute 30 g de poly éthylène glycol 400 (commercialisé sous la dénomination Lutrol E 400). On ajoute avec précaution quelques gouttes de solution d'hydroxyde de sodium à 30 %, à la solution acide (pH 4,7) pour obtenir un pH de 5,5. On complète la solution avec de l'eau purifiée à 200 g. La solution est alors introduite dans des flacons sous pression de 15 ml. On ferme le flacon avec le gicleur et on injecte de l'azote jusqu'à ce que la pression gazeuse à l'intérieur s'élève à 7 bars. On place alors la valve que l'on sertit sur l'embout du gicleur.

### EXEMPLE 2

### Soluté de Kétoprofène, sel de Trométhamine

On dissout 6,65 g de sel de Trométhamine de Kétoprofène dans 80 g d'éthanol à 50 %. On ajoute 12 g de poly éthylène glycol 400 et on ajuste le pH du milieu à 5,5 par addition d'une solution d'acide chlorhydrique à 1 %. On complète la solution à 200g avec de l'eau de roses. On répartit après homogénéisation la solution hydroalcoolique dans des flacons pressurisés de 15 ml. On obture le flacon par insertion du gicleur monté sur le tube plongeur. On injecte de l'azote jusqu'à ce que la pression interne s'élève à 7 bars. On dispose alors la valve que l'on sertit sur l'embout du gicleur.

### EXEMPLE III

### Soluté de Kétoprofène avec agent pénétrant

On prépare une solution prête à la pulvérisation, de formule :

| | |
|---|---|
| - Kétaprofène | 2,5 % |
| - Lutrol E 400 | 10 % |
| - Huile essentielle de Lavandin | 0,1 % |
| - Solkétal | 5 % |
| - Alcool 95-96 % | 40 % |
| - Eau purifiée qsp | 100 % |
| - Hydroxyde de sodium à 30 % qsp | pH 5,5 |

Les compositions selon l'invention trouvent une utilisation dans le traitement des phénomènes douloureux ou inflammatoires de l'appareil locomoteur notamment en cas de tendinites ou dans la traumatologie.

## Revendications

1. Compositions pharmaceutiques liquides contenant un agent anti-inflammatoire, **caractérisées en ce qu'**elles renferment du kétoprofène sous forme acide ou sous forme salifiée, dispersée dans un alcanol ayant de 1 à 6 atomes de carbone dilué par de l'eau, un polyéthylène glycol 400 ou 600 et un agent d'ajustement à pH neutre et **en ce que** les compositions sont réparties sous pression de gaz inerte en flacons pressurisés.

2. Compositions pharmaceutiques à action anti-inflammatoire selon la revendication 1, dans lesquelles l'agent anti-inflammatoire est le kétoprofène sous la forme (R) ou (S).

3. Compositions pharmaceutiques à action anti-inflammatoire selon la revendication 1, dans lesquelles le kétoprofène est sous forme salifiée.

4. Compositions pharmaceutiques à action anti-inflammatoire selon la revendication 1, dans lesquelles le kétoprofène est sous forme de sel avec une base minérale.

5. Compositions pharmaceutiques à action anti-inflammatoire selon la revendication 1, dans lesquelles le kétoprofène est sous forme de sel avec une base organique.

6. Compositions pharmaceutiques selon la revendication 1, dans lesquelles l'alcanol est choisi parmi le méthanol, l'éthanol, le propanol, l'isopropanol, les n et i-butanols, le terbutanol, le néopentanol ou le n-hexanol.

7. Compositions pharmaceutiques selon l'une des revendications précédentes, comportant en outre un agent de pénétration.

8. Compositions pharmaceutiques selon l'une des revendications précédentes dans lesquelles l'agent de pénétration est choisi parmi le solkétal, l'azone, le 2-n nonyldioxolane, le méthylal, le monocaprylate de propylène glycol et l'octyl-2 dodécanol.

9. Compositions pharmaceutiques selon l'une des revendications précédentes dans lesquelles la solution d'agent anti-inflammatoire est ajustée à un pH voisin de 5 à 5,5.

10. Compositions pharmaceutiques selon la revendication 1 dans lesquelles le gaz inerte est de l'azote, le protoxyde d'azote, le gaz carbonique ou un hydrocarbure.

11. Compositions pharmaceutiques selon la revendication 1 ou 10 dans lesquelles le gaz inerte est présent sous une pression de préférence égale à 7 bars.

## Patentansprüche

1. Flüssige pharmazeutische Zusammensetzungen, die einen entzündungshemmenden Wirkstoff enthalten, **dadurch gekennzeichnet, dass** sie Ketoprofen in saurer Form oder versalzter Form, dispergiert in einem mit Wasser verdünnten Alkanol mit 1 bis 6 Kohlenstoffatomen, ein Polyethylenglykol 400 oder 600 sowie ein Mittel zum Einstellen auf neutralen pH-Wert enthalten und dass die Zusammensetzungen unter Inertgasdruck in Druckfläschchen verteilt werden.

2. Pharmazeutische Zusammensetzungen mit entzündungshemmender Wirkung nach Anspruch 1, bei denen der entzündungshemmende Wirkstoff Ketoprofen in (R)-oder (S)-Form ist.

3. Pharmazeutische Zusammensetzungen mit entzündungshemmender Wirkung nach Anspruch 1, bei denen Ketoprofen in versalzter Form vorliegt.

4. Pharmazeutische Zusammensetzungen mit entzündungshemmender Wirkung nach Anspruch 1, bei denen Ketoprofen in Form von Salz mit einer mineralischen Base vorliegt.

5. Pharmazeutische Zusammensetzungen mit entzündungshemmender Wirkung nach Anspruch 1, bei denen Ketoprofen in Form von Salz mit einer organischen Base vorliegt.

6. Pharmazeutische Zusammensetzungen nach Anspruch 1, bei denen das Alkanol aus Methanol, Ethanol, Propanol, Isopropanol, den n- und i-Butanolen, tert-Butanol, neo-Pentanol oder n-Hexanol ausgewählt ist.

7. Pharmazeutische Zusammensetzungen nach einem der vorhergehenden Ansprüche, ferner umfassend ein Penetrationsmittel.

8. Pharmazeutische Zusammensetzungen nach einem der vorhergehenden Ansprüche, bei denen das Penetrationsmittel aus Solketal, Azon, 2-n-Nonyldioxolan, Methylal, Propylenglykol-Monocapryplat und Octyl-2 dodecanol ausgewählt ist.

9. Pharmazeutische Zusammensetzungen nach einem der vorhergehenden Ansprüche, bei denen die entzündungshemmende Wirkstofflösung auf einen pH-Wert nahe 5 bis 5,5 eingestellt wird.

10. Pharmazeutische Zusammensetzungen nach Anspruch 1, bei denen das Inertgas Stickstoff, Distickstoffmonoxid, Kohlendioxid oder ein Kohlenwasserstoff ist.

11. Pharmazeutische Zusammensetzungen nach Anspruch 1 oder 10, bei denen das Inertgas unter einem Druck von vorzugsweise gleich 7 bar vorliegt.

## Claims

1. Liquid pharmaceutical compositions containing an anti-inflammatory agent, **characterized in that** they contain ketoprofen in acid or salt form, dispersed in an alkanol having 1 to 6 carbon atoms diluted in water, a polyethylene glycol 400 or 600 and an agent to adjust the pH to neutral and also **in that** the compositions are divided up and placed under pressure of an inert gas in pressurized vials.

2. Pharmaceutical compositions having an anti-inflammatory action according to claim 1, in which the anti-inflammatory agent is ketoprofen in the (R) or (S) form.

3. Pharmaceutical compositions having an anti-inflammatory action according to claim 1, in which the ketoprofen is in a salt form.

4. Pharmaceutical compositions having an anti-inflammatory action according to claim 1, in which the ketoprofen is in the form of a salt with an inorganic base.

5. Pharmaceutical compositions having an anti-inflammatory action according to claim 1, in which the ketoprofen is in a form of a salt with an organic base.

6. Pharmaceutical compositions according to claim 1, in which the alkanol is chosen among methanol, ethanol, propanol, isopropanol, n-and i-butanols, t-butanol, neopentanol or n-hexanol.

7. Pharmaceutical compositions according to one of the preceding claims, further comprising a penetration agent.

8. Pharmaceutical compositions according to one of the preceding claims in which the penetration agent is chosen among solketal, azone, 2-n nonyldioxolane, methylal, propylene glycol monocaprylate and octyl-2-dodecanol.

9. Pharmaceutical compositions according to one of the preceding claims in which the solution of the anti-inflammatory agent is adjusted in order to have a pH close to 5 to 5.5.

10. Pharmaceutical compositions according to claim 1 in which the inert gas is nitrogen, nitrous oxide, carbon dioxide or a hydrocarbon.

11. Pharmaceutical compositions according to claim 1 or 10 in which the inert gas is present under a pressure preferably equal to 7 bars.
